(19) Europäisches Patentamt
European Patent Office
Office européen des brevets

(11) Publication number: **0 095 104**
**B1**

# (12) EUROPEAN PATENT SPECIFICATION

(45) Date of publication of patent specification: **17.12.86**

(51) Int. Cl.$^4$: **C 07 D 401/04** // C07D213/65

(21) Application number: **83104716.2**

(22) Date of filing: **13.05.83**

**(54) Process for the preparation of pyridyl and quinolyl imidazolinones.**

(30) Priority: **25.05.82 US 381817**

(43) Date of publication of application:
**30.11.83 Bulletin 83/48**

(45) Publication of the grant of the patent:
**17.12.86 Bulletin 86/51**

(84) Designated Contracting States:
**BE CH DE FR GB IT LI NL SE**

(56) References cited:
**EP-A-0 041 623**

**SYNTHESIS, vol. 7, June 1975, pp. 358-375, Stuttgart, DE; E.V.BROWN: "The Willgerodt reaction"**

**JUSTUS LIEBIGS ANNALEN DER CHEMIE, 1975, pages 1994-2005, Weinheim, DE; H.HAGEN et al.: "Neue Synthesen mit elementarem Schwefel. Darstellung von 2-(1,3-Diaza-2-cycloalken-2-yl)th iophenolen und ihre Umwandlung zu 1,4-Benzothiazepinen und 1,3,4-Benzothiadiazepinen"**

(73) Proprietor: **AMERICAN CYANAMID COMPANY**
**1937 West Main Street P.O. Box 60**
**Stamford Connecticut 06904 (US)**

(72) Inventor: **Wepplo, Peter John**
**45 Wilton Street**
**Princeton, NJ 08540 (US)**

(74) Representative: **Wächtershäuser, Günter, Dr.**
**Tal 29**
**D-8000 München 2 (DE)**

**EP 0 095 104 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Summary of the Disclosure

In accordance with the process of the invention, formula (I) pyridyl and quinolyl imidazolinones can be prepared by a procedure involving heating, from about 1 to 20 equivalents and preferably at least 1.5 equivalents and most preferably 1.5 to 10.0 equivalents, of a 5-substituted formula (II) 2-picoline with at least 1 equivalent of an appropriate formula (III) aminocarboxamide in the presence of at least 3 equivalents and preferably 3 to 5 equivalents of sulfur. In this reaction, at least a 3 to 1 ratio of sulfur to aminocarboxamide and at least 1 and preferably 1.5 equivalents of the picoline is essential for good product yields. The reaction may optionally be conducted in a solvent which has a boiling point within the reaction temperature range. With regard to the picoline, it should be recognized that the compound, while being a reactant, may also be used as a solvent for the reaction and used in large excess. The reaction mixture is boiled, generally at a temperature from 100 to 250°C, and the vapors therefrom either distilled off or passed through a column packed with molecular sieves to remove any water formed during reaction. The dried condensed reaction mixture is then returned to the reaction vessel, and heating of the mixture is continued for several hours. Thereafter, the mixture is cooled, dissolved in an organic solvent such as ethyl acetate, diethyl ether or the like and filtered. The filtrate is extracted with an aqueous mineral acid such as sulfuric acid or hydrochloric acid, and the aqueous phase treated with aqueous base to liberate the formula (I) imidazolinone. If the product precipitates as a solid, it is recovered by filtration; if as an oil, it is extracted into a solvent such as dichloromethane, ether, and the like, and the product purified by conventional methods. The reaction may be graphically illustrated as follows:

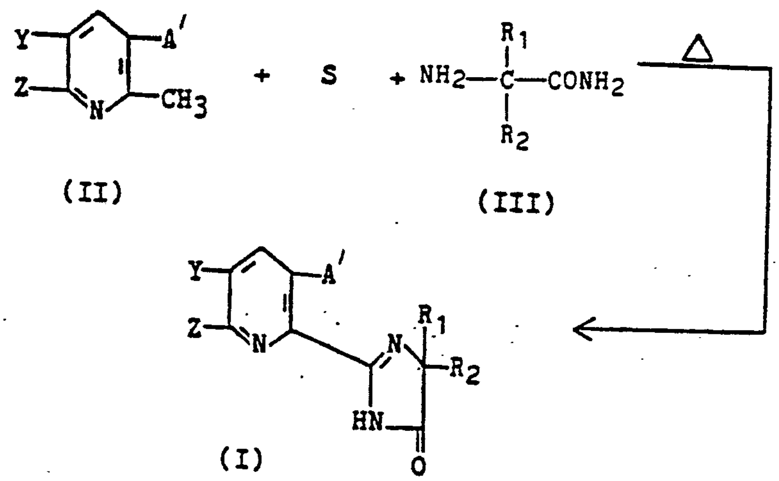

(II) (III) (I)

wherein Z is hydrogen; Y is hydrogen, chlorine, fluorine $C_1$—$C_6$ alkyl, $C_1$—$C_6$ alkoxy, phenyl or substituted phenyl; A′ is hydrogen, primary or secondary $C_1$—$C_6$ alkyl or $COOR_3$ where $R_3$ is $C_1$—$C_{12}$ alkyl and $R_1$ and $R_2$ are $C_1$—$C_4$ alkyl or when taken together they may represent $C_3$—$C_6$ cycloalkyl optionally substituted with methyl and when $R_1$ and $R_2$ are not the same, the optical isomers thereof; and when taken together, Y and Z may form a ring in which YZ is

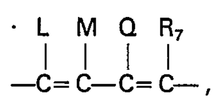

where L, M, Q and $R_7$ each represent members selected from the group consisting of hydrogen, chlorine, fluorine, methyl, methoxy or phenyl; when A′ is hydrogen or $COOR_3$ it may be converted to COOH. The process is thus highly effective for preparing variously substituted formula (I) imidazolinones from 2-picolines and substituted quinaldines.

Preparation of the 5-alkoxy-2-picoline employed in the above reaction for the preparation of 2-(5-alkoxy-2-pyridyl)-5,5-di(lower)alkyl-2-imidazolin-4-one compounds is achieved by reacting 5-hydroxy-2-methylpyridine with an equivalent amount of a $C_1$—$C_4$ alkyl iodide in the presence of sodium hydride and a solvent such as dry dimethylformamide at a temperature of from 0 to 30°C under a blanket of inert gas such as nitrogen.

As indicated above, the process of the invention is unique since it provides an unexpectedly effective method for the conversion, in a single step, of a substituted 2-picoline or substituted quinaldine to the herbicidially active pyridyl and quinolyl imidazolinones represented by formula (I) and for intermediates for herbically active acids of formula (I) in which A′=COOH.

The closest art process of which we are aware is commonly referred to as the Willgerodt reaction.

It is apparent from reviews of the Willgerodt reaction [e.g. Synthesis, 358 (1975)], that the Kindler

modification utilizes dry amines, ammonia, primary and secondary amines, to give thioamides. This reaction of 2-picoline, sulfur and aniline is described and can be illustrated as follows:

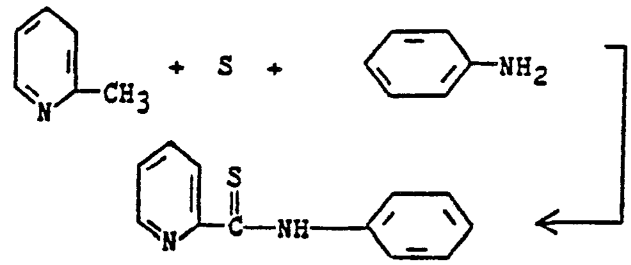

Clearly, the above process of Willgerodt does not suggest or render obvious the process of our invention which involves the discovery that when the amine in the above process, is replaced by an α-aminocarboxamide of the formula:

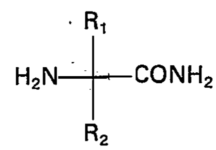

wherein $R_1$ and $R_2$ are $C_1$—$C_4$ alkyl groups, the reaction produces directly, in one step, the heterocyclic formula (I) imidazolinones, which are herbicides and/or intermediates for the preparation of herbicides.

As will be evident from the data provided below, the above formula (I) pyridyl and quinolyl imidazolinones are highly effective herbicidal agents or intermediates for the preparation of highly effective herbicides, useful for the control of a wide variety of annual and perennial monocotyledonous and dicotyledonous plants. They are also effective as aquatic herbicides. The compounds may be used for the control of undesirable plant species by applying to the foliage of the plants or to soil or water containing seeds or other propagating organs of the plants, a herbicidally effective amount of a formula (I) pyridyl or quinolyl imidazolinone. In practice the compounds are generally effective as herbicidal agents when applied at a rate sufficient to provide from about 0.016 to 10 kg/ha and preferably 0.016 to 4 kg/ha.

Among the compounds prepared by the process of this invention, which are effective as herbicides or are intermediates thereof are: 5-isopropyl-5-methyl-2-(2-pyridyl)-2-imidazolin-4-one; 5-isopropyl-5-methyl-2-(3-methyl-2-pyridyl)-2-imidazolin-4-one; ethyl 2-(5-isopropyl-5-methyl-4-oxo-2-imidazolin-2-yl)nicotinate; ethyl-(5-isopropyl-5-methyl-4-oxo-2-imidazolin-2-yl)-3-quinolinecarboxylate.

These herbicidal compounds are described and exemplified in the European Patent Application publication number 0041623 published December 16, 1981 Bulletin 81/50, Application number 81103638.3 of Marinus Los.

In order to facilitate a further understanding of the invention, the following examples are presented primarily for the purpose of illustrating certain more specific details thereof. The invention is not to be deemed limited thereby. Unless otherwise noted, all parts are by weight.

## Example 1

Preparation of the intermediate 5-ethoxy-2-methylpyridine

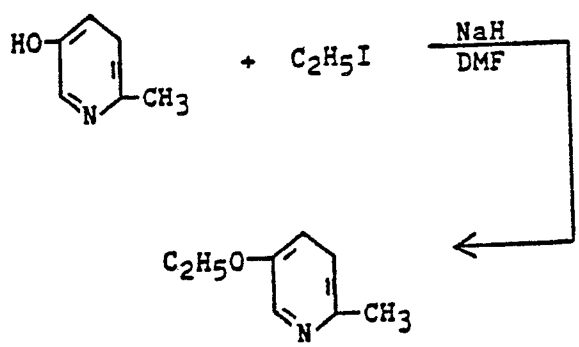


To a stirred suspension of 16.6 g sodium hydride in 500 ml dry dimethylformamide (DMF) under nitrogen at 0°C is added 90 g 5-hydroxy-2-methylpyridine at such a rate that the temperature remains between 0—5°C. When gas evolution ceases, 73.8 ml ethyl iodide in 100 ml DMF is added dropwise. After stirring at room temperature overnight, the mixture is diluted with water and extracted with ether. The

ether extracts are washed with brine, dried and concentrated. This oil is distilled to give 62.5 g of 5-ethyl-2-methylpyridine, bp 89—91°C at 12 mm.

Using the above procedure but substituting isopropyl iodide for ethyl iodide there is obtained 5-isopropoxy-2-methylpyridine, bp 95—100°C/0.15 mm.

Example 2

Preparation of 2-(5-ethoxy-2-pyridyl)-5-isopropyl-5-methyl-2-imidazolin-4-one

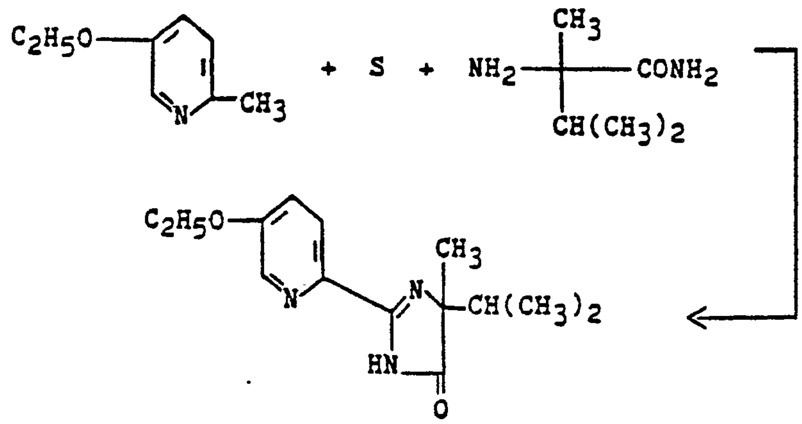

A mixture of 60.5 g 5-ethoxy-2-picoline, 38.3 g 2-amino-2,3-dimethylbutyramide and 28.25 g sulfur is heated with stirring under nitrogen. At 160°C a liquid boils which is condensed and returned to the flask through a tube packed with molecular sieves to absorb water formed in the reaction. The temperature of the mixture slowly rises to 185°C and is held there for 2.5 hours. The mixture is cooled, dissolved in 500 ml ethyl acetate and filtered. The filtrate is extracted with 6 × 100 ml portions of 2N hydrochloric acid. The aqueous phases are combined, the pH adjusted to 7 with 50% aqueous sodium hydroxide. A solid precipitate forms which is collected. The mother liquor is extracted with methylene chloride, the extracts dried and concentrated. The residue is triturated with ether-hexane to give a crystalline solid which is removed and thoroughly washed with hexane and dried. Both solids are combined to give 23.7 g product. Two recrystallizations of a sample from methylene chloride-hexane gives analytically pure 2-(5-ethoxy-2-pyridyl)-5-isopropyl-5-methyl-2-imidazolin-4-one, mp 143—145°C.

Using essentially the same procedure described above but substituting the appropriate substituted α-picoline or quinaldine for 5-ethoxy-2-picoline. The following imidazolinones are prepared:

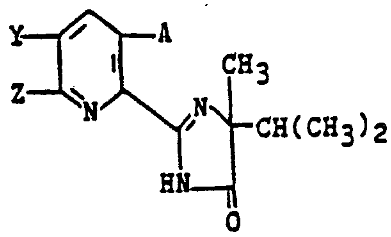

| Substituent | mp·C |
|---|---|
| none | 90—93 |
| 5-$C_2H_5$ | 67—71 |
| 5-$OCH_3$ | 166—167.5 |
| 5-$C_6H_5$ | 150—151.5 |
| 3-$CH_3$ | 93—96 |
| 3-$COOC_2H_5$ | 72—75 |
| 5-$OCH(CH_3)_2$ | 126—128 |
| 2-$C_2H_5$ | 73—75 |
| 5-$C_2H_5$ | 64—66 |
| | $[\alpha]_D^{20} = +5.3°$ |
| | (C=0.05 g/ml THF) |

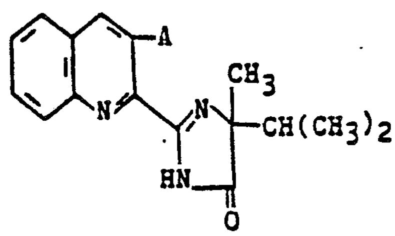

| Substituent | mp°C |
|---|---|
| *none | 143—144 |
| 3-$COOC_2H_5$ | 146—147.5 |
| *none | 113.5—115.5, |
| | $[\alpha]_D^{25} = +18.75°$ |
| | (C=0.027 g/ml EtOH) |

*optical isomers

Example 3

Preparation of 5-ethoxy-2-(5-isopropyl-5-methyl-4-oxo-2-imidazolin-2-yl)nicotinic acid

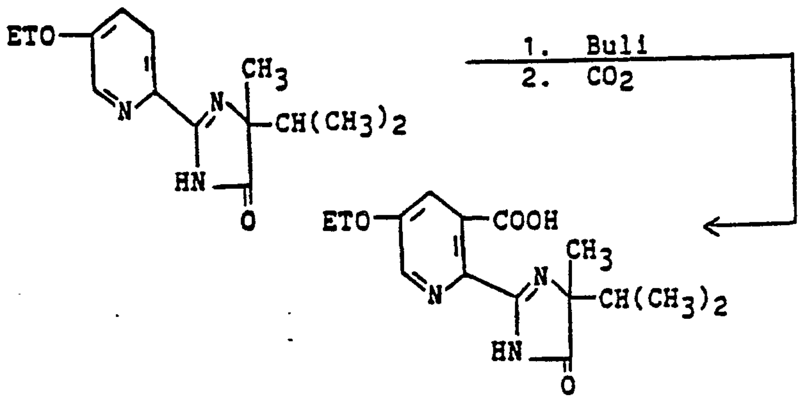


To a stirred solution containing 13.5 g ethoxypyridine derivative in 250 ml dry THF at −70°C under nitrogen is added dropwise 65 ml of 1.8 m solution of butyl lithium in hexane without allowing the temperature to rise above −65°C. After a further 2 hours at −70°C, 15 ml butyl lithium solution is added dropwise and stirring continued for 0.5 hours. Solid carbon dioxide in excess is then added to the reaction mixture and the mixture allowed to attain room temperature. The solvent is removed, the residue taken up in 250 ml water, the pH adjusted to 8 with 6N $H_2SO_4$ and extracted with $CH_2Cl_2$. The organic phase is discarded. The pH of the aqueous phase is adjusted to 3. The precipitate (3.8 g) is collected and dried. The filtrate is extracted with $CH_2Cl_2$. TLC of the extract showed the presence of some starting material. The organic phase is extracted with 4 × 100 ml saturated sodium bicarbonate solution. These are combined, acidified with concentrated $H_2SO_4$ to pH 3 to give a solid (4.5 g) identical with the solid collected above. A sample recrystallized from acetonitrile has mp 140—144°C (decomposes) and is analytically pure 5-ethoxy-2-(5-isopropyl-5-methyl-4-oxo-2-imidazolin-2-yl)nicotinic acid.

Using essentially the same procedure described above but substituting the appropriately substituted 5-isopropyl-5-methyl-2-(2-pyridyl)- or -2-(2-quinolyl)-2-imidazolin-4-one for 2-(5-ethoxy-2-pyridyl)-5-isopropyl-5-methyl-2-imidazolin-4-one, the following nicotinic or quinoline-3-carboxylic acids are prepared.

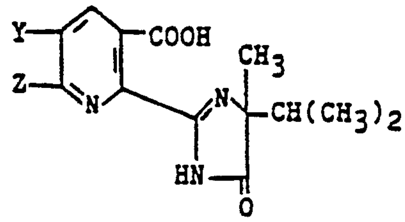

| Y | Z | mp°C |
|---|---|---|
| $C_2H_5$ | H | 172—175.0 |
| $OCH_3$ | H | 166—167.5 |
| $C_6H_5$ | H | 150—151.5 |
| $OCH(CH_3)_2$ | H | 83—85 |
| H | H | 170—172.5 |
| $C_2H_5$ | H | 121—123, $[\alpha]_D^{20} = +13.4°$ (C=0.0908 g/ml THF) |
| * 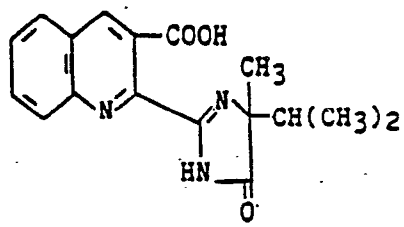 | | 219—222.0 |
| * 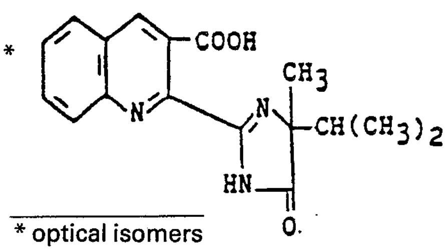 | | 228—236.5, $[\alpha]_D^{25} = +28.3°$ (C=0.0105 g/ml $CH_2Cl_2$) |

* optical isomers

Example 4

Preparation of 2-(5-isopropoxy-2-pyridyl)-5-isopropyl-5-methyl-2-imidazolin-4-one

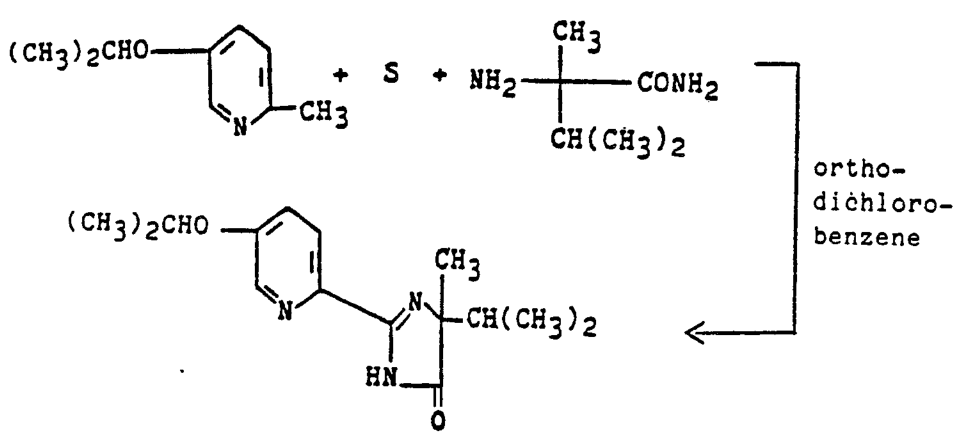


A mixture containing 1.97 kg of 5-isopropoxy-2-picoline, 2.09 kg of 2-amino-2,3-dimethylbutyramide and 1.78 kg of sulfur in 7.5 liters of *ortho*-dichlorobenzene is heated to 180°C while stirring under nitrogen.

After stirring at 180°C for 10 hours, the mixture is cooled to room temperature and clarified by filtration. The filtrate is extracted with 19 liters of 4.5% aqueous hydrochloric acid and the aqueous layer separated off.

Methylene chloride (10 liters) is added to the aqueous extract and the pH of the mixture adjusted to pH 6.5 with 50% aqueous sodium hydroxide. The organic phase is separated and the aqueous layer extracted with additional methylene chloride (2 liters). The organic extracts are combined, dried over anhydrous $MgSO_4$, filtered and concentrated on a rotary evaporator. The residue is slurried with heptanes for one hour and the resulting solid filtered off and dried to yield 904 g of 2-(5-isopropoxy-2-pyridyl)-5-isopropyl-5-methyl-2-imidazolin-4-one.

Example 5

Preparation of 2-(5-ethyl-2-pyridyl)-5-isopropyl-5-methyl-2-imidazolin-4-one

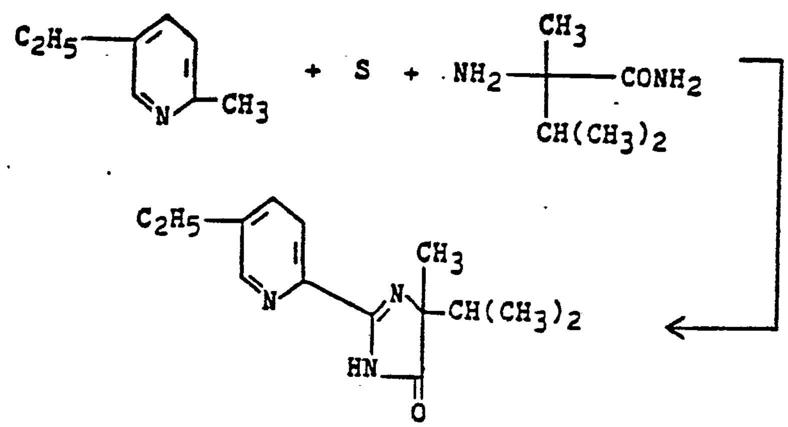

A mixture of 183.8 g 5-ethyl-2-picoline, 40.0 g 2-amino-2,3-dimethylbutyramide and 29.6 sulfur is heated to 180—190°C with stirring under nitrogen. The temperature of the mixture slowly rises to 180—190°C while low boiling components are collected in a Dean Stark Trap, and held there for 3 hours. The mixture is cooled and the excess picoline removed by distillation under reduced pressure., dissolved in 200 mL ethyl acetate and filtered. The filtrate is extracted with 6 × 100 mL portions of 2N hydrochloric acid. The aqueous phases are combined, the pH adjusted to 8 with 50% aqueous sodium hydroxide. The solution is extracted with ethyl acetate 5 × 150 mL, the extracts combined, treated with activated charcoal, filtered and concentrated. This affords 56.7 g (75% yield) of 2-(5-ethyl-2-pyridyl-5-isopropyl-5-methyl-2-imidazolin-4-one, as a red gum which is 94.9% pure.

Example 6

Preparation of 2-(5-isopropyl-5-methyl-4-oxo-imidazolin-2-yl)nicotinic acid

A solution of potassium *tertiary*-butoxide (2.8 g, 0.025 mol) in dimethylformamide (DMF 10 mL) is added dropwise to a stirred solution of 2-(3-methyl-2-pyridyl)-5-isopropyl-5-methyl-2-imidazolin-4-one (1.2 g 0.005 mol) in dry DMF while bubbling oxygen through the reaction mixture. Upon completion of the addition the mixture is stirred at 40—50°C for three hours, then it is cooled, clarified by filtration, and diluted with water (50 mL). The pH of the aqueous solution is adjusted to pH 2 and the product extracted into ethyl acetate.

The organic solution is dried over $MgSO_4$, filtered and the solvent removed under reduced pressure to yield 0.76 g (57% yield) of 2-(5-isopropyl-5-methyl-4-oxo-2-imidazolin-2-yl)nicotinic acid, mp 166—167°C.

**Claims**

1. A method for the preparation of a compound of the formula:

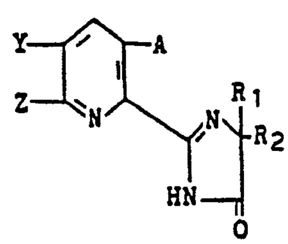

wherein Z is hydrogen; Y is hydrogen, chlorine, fluorine, $C_1$—$C_6$ alkyl, $C_1$—$C_6$ alkoxy, phenyl or substituted phenyl; A is hydrogen, $C_1$—$C_6$ primary or secondary alkyl, COOH or $COOR_3$ where $R_3$ is $C_1$—$C_{12}$ alkyl and $R_1$ and $R_2$ are $C_1$—$C_4$ alkyl or when taken together they may represent $C_3$—$C_6$ cycloalkyl optionally substituted

with methyl and when $R_1$ and $R_2$ are not the same, the optical isomers thereof; and when taken together, Y and Z may form a ring in which YZ is

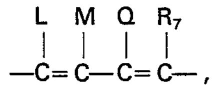

where L, M, Q and $R_7$ each represent members selected from the group consisting of hydrogen, chlorine, fluorine, methyl, methoxy or phenyl; comprising as a part of the synthesis sequence reacting at a temperature of from 100 to 250°C from 1 to 20 equivalents of a compound of the formula:

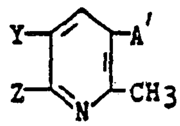

wherein Y and Z are as described above and A′ is the same as A with the exception of COOH; with at least 3 equivalents of sulfur and about 1 equivalent of an aminocarboxamide of the formula:

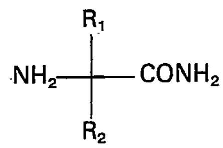

wherein $R_1$ and $R_2$ are as described above; and when desired when A′ is hydrogen or $—COOR_3$ converting to COOH.

2. A method according to Claim 1 wherein 1.5 to 10 equivalents of α-picoline is reacted with 3 to 5 equivalents of sulfur and 1 equivalent of aminocarboxamide.

3. A method according to Claim 1 wherein A and Z are hydrogen; Y is $C_1—C_6$ alkoxy; $R_1$ is methyl and $R_2$ is isopropyl.

4. A method according to Claim 1 wherein Y is $C_1—C_6$ alkyl; A and Z are hydrogen; $R_1$ is methyl and $R_2$ is isopropyl.

5. A method according to Claim 1 wherein Y and Z are taken together and represent

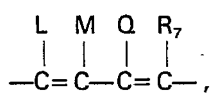

where L, M, Q and $R_7$ each represent members selected from the group consisting of hydrogen, chlorine, fluorine, methyl, methoxy or phenyl; A is as described in Claim 1; $R_1$ is methyl and $R_2$ is isopropyl.

6. A method according to Claim 1 wherein Y is phenyl; Z is hydrogen; A is as described in Claim 1; $R_1$ is methyl and $R_2$ is isopropyl.

7. A method according to Claim 1 wherein Y is ethyl, A and Z are hydrogen, R is methyl and $R_2$ is isopropyl.

8. A method according to Claim 1 wherein Y and Z are hydrogen, A is $CH_3$, $R_1$ is $CH_3$, $R_2$ is $CH(CH_3)_2$.

9. A method according to Claim 1 wherein Y are Z are hydrogen, A is $COOR_3$, $R_1$ is $CH_3$, $R_2$ is $CH(CH_3)_2$ and $R_3$ is as described in Claim 1.

10. A method according to Claim 1 wherein 1,5 equivalents of α-picoline or quinaldine is heated with 3 equivalents of sulfur and 1 equivalent of the α-aminocarboxamide to obtain the desired pyridyl or quinolyl imidazolinone.

**Patentansprüche**

1. Verfahren zur Herstellung einer Verbindung der Formel

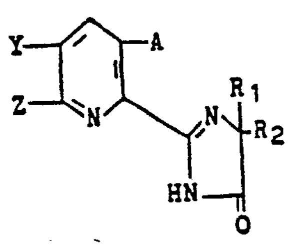

wobei Z für Wasserstoff steht; Y für Wasserstoff, Chlor, Fluor, $C_1—C_6$-Alkyl, $C_1—C_6$-Alkoxy, Phenyl oder

substituiertes Phenyl steht; A für Wasserstoff, $C_1$—$C_6$ primäres oder sekundäres Alkyl, COOH oder $COOR_3$ steht, wobei $R_3$ für $C_1$—$C_{12}$-Alkyl steht und $R_1$ und $R_2$ für $C_1$—$C_4$-Alkyl stehen oder zusammengefaßt $C_3$—$C_6$-Cycloalkyl bedeuten können, das gegebenenfalls mit Methyl substituiert ist, und falls $R_1$ und $R_2$ nicht gleich sind, die optischen Isomeren derselben; wobei Y und Z zusammengefaßt einen Ring bilden können, bei dem YZ für

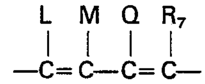

steht, wobei L, M, Q und $R_7$ jeweils ausgewählt sind aus der Gruppe Wasserstoff, Chlor, Fluor, Methyl, Methoxy oder Phenyl; umfassend als Teil der Synthesesequenz die Umsetzung von 1 bis 20 Äquivalenten einer Verbindung der Formel

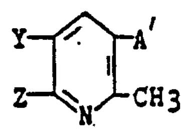

wobei Y und Z die oben angegebene Bedeutung haben und A' die gleiche Bedeutung hat wie A mit Ausnahme von COOH, bei einer Temperatur von 100 bis 250°C mit mindestens 3 Äquivalenten Schwefel und etwa 1 Äquivalent eines Aminocarboxamids der Formel

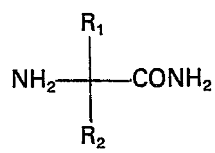

wobei $R_1$ und $R_2$ die oben angegebene Bedeutung haben und wobei man gegebenenfalls dann, wenn A' für Wasserstoff oder —$COOR_3$ steht, eine Umwandlung in COOH vornimmt.

2. Verfahren gemäß Anspruch 1, wobei 1,5 bis 10 Äquivalente α-Picolin umgesetzt werden mit 3 bis 5 Äquivalenten Schwefel und 1 Äquivalent aminocarboxamid.

3. Verfahren gemäß Anspruch 1, wobei A und Z fur Wasserstoff stehen; Y für $C_1$—$C_6$-Alkoxy steht; $R_1$ fur Methyl steht und $R_2$ für Isopropyl steht.

4. Verfahren gemäß Anspruch 1, wobei Y für $C_1$—$C_6$-Alkyl steht; A und Z für Wasserstoff stehen; $R_1$ für Methyl steht und $R_2$ für Isopropyl steht.

5. Verfahren gemäß Anspruch 1, wobei Y und Z zusammegefaßt sind und für

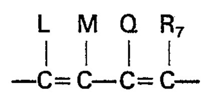

stehen, wobei L, M, Q und $R_7$ jeweils ausgewählt sind aus der Gruppe Wasserstoff, Chlor, Fluor, Methyl, Methoxy oder Phenyl; A die in Anspruch 1 angegebene Bedeutung hat; $R_1$ für Methyl steht und $R_2$ für Isopropyl steht.

6. Verfahren gemäß Anspruch 1, wobei Y für Phenyl steht; Z für Wasserstoff steht; A die in Anspruch 1 angegebene Bedeutung hat; $R_1$ für Methyl steht und $R_2$ für Isopropyl steht.

7. Verfahren gemäß Anspruch 1, wobei Y für Äthyl steht, A und Z für Wasserstoff stehen; $R_1$ für Methyl steht und $R_2$ für Isopropyl steht.

8. Verfahren gemäß Anspruch 1, wobei Y und Z für Wasserstoff stehen; A für $CH_3$ steht, $R_1$ für $CH_3$ steht, $R_2$ für $CH(CH_3)_2$ steht.

9. Verfahren gemäß Anspruch 1, wobei Y und Z für Wasserstoff stehen, A für $COOR_3$ steht, $R_1$ für $CH_3$ steht, $R_2$ für $CH(CH_3)_2$ und $R_3$ die in Anspruch 1 angegebene Bedeutung hat.

10. Verfahren gemäß Anspruch 1, wobei 1,5 Äquivalente α-Picolin oder Quinaldin erhitzt werden mit 3 Äquivalenten Schwefel und 1 Äquivalent des α-Aminocarboxamids, um das angestrebte Pyridyl- oder Quinolyimidazolinon zu erhalten.

**Revendications**

1. Procédé pour la préparation d'un composé de formule:

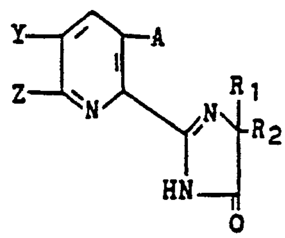

dans laquelle Z est un hydrogène; Y est un hydrogène, un chlore, un fluor, un alkyle en $C_1$—$C_6$, un alcoxy en $C_1$—$C_6$, un phényle ou un phényle substitué; A est un hydrogène, un alkyle primaire ou secondaire en $C_1$—$C_6$, COOH ou $COOR_3$ où $R_3$ est un alkyle en $C_1$—$C_{12}$ et $R_1$ et $R_2$ sont un alkyle en $C_1$—$C_4$ ou pris ensemble peuvent représenter un cycloalkyle en $C_3$—$C_6$ éventuellement substitué par un méthyle et, lorsque $R_1$ et $R_2$ sont différents, les isomères optiques correspondants; et lorsqu'ils sont pris ensemble Y et Z peuvent former un cycle dans lequel YZ est

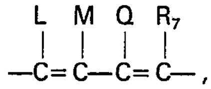

où L, M, Q et $R_7$ représentent chacun des composants choisis dans le groupe constitué par un hydrogène, un chlore, un fluor, un méthyle, un méthoxy ou un phényle; comprenant comme partie de la séquence de synthèse la réaction à une température de 100 à 250°C de 1 à 20 équivalents d'un composé de formule:

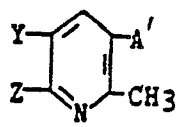

dans laquelle Y et Z sont comme décrit ci-dessus et A' est comme A à l'exception de COOH; avec au moins 3 équivalents de soufre et environ 1 équivalent d'un aminocarboxamide de formule:

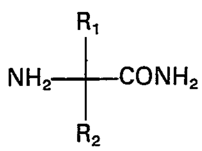

dans laquelle $R_1$ et $R_2$ sont comme décrit ci-dessus; et lorsqu'on le désire, lorsque A' est un hydrogène ou —$COOR_3$, la conversion en COOH.

2. Procédé selon la revendication 1 où l'on fait réagir 1,5 à 10 équivalents d'α-picoline avec 3 à 5 équivalents de soufre et 1 équivalent d'aminocarboxamide.

3. Procédé selon la revendication 1 dans lequel A et Z sont un hydrogène; Y est un alcoxy en $C_1$—$C_6$; $R_1$ est un méthyle et $R_2$ est un isopropyle.

4. Procédé selon la revendication 1 dans lequel Y est un alkyle en $C_1$—$C_6$; A et Z sont un hydrogène; $R_1$ est un méthyle et $R_2$ est un isopropyle.

5. Procédé selon la revendication 1 dans lequel Y et Z sont pris ensemble et représentent

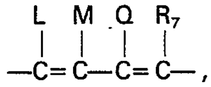

où L, M, Q et $R_7$ représentent chacun des composants choisis dans le groupe constitué par un hydrogène, un chlore, un fluor, un méthyle, un méthoxy ou un phényle; A est comme décrit dans la revendication 1; $R_1$ est un méthyle et $R_2$ est un isopropyle.

6. Procédé selon la revendication 1 dans lequel Y est un phényle, Z est un hydrogène, A est comme décrit dans la revendication 1; $R_1$ est un méthyle et $R_2$ est un isopropyle.

7. Procédé selon la revendication 1 dans lequel Y est un éthyle, A et Z sont un hydrogène, R est un méthyle et $R_2$ est un isopropyle.

8. Procédé selon la revendication 1 dans lequel Y et Z sont un hydrogène, A est $CH_3$, $R_1$ est $CH_3$ et $R_2$ est $CH(CH_3)_2$.

9. Procédé selon la revendication 1 dans lequel Y et Z sont un hydrogène, A est $COOR_3$, $R_1$ est $CH_3$, $R_2$ est $CH(CH_3)_2$ et $R_3$ est comme décrit dans la revendication 1.

10. Procédé selon la revendication 1 dans lequel on chauffe 1,5 équivalent d'α-picoline ou de quinaldine avec 3 équivalents de soufre et 1 équivalent de l'α-aminocarboxamide pour obtenir la pyridyl- ou quinolylimidazolinone requise.